(19) Europäisches Patentamt / European Patent Office / Office européen des brevets

(11) EP 4 243 846 B1

(12) **EUROPEAN PATENT SPECIFICATION**

(45) Date of publication and mention of the grant of the patent:
**23.09.2026 Bulletin 2026/39**

(21) Application number: **21811536.8**

(22) Date of filing: **12.11.2021**

(51) International Patent Classification (IPC):
***A61K 35/747*** (2015.01) ***A61K 35/744*** (2015.01)
***A61K 31/702*** (2006.01) ***A61P 31/14*** (2006.01)
***A61P 1/00*** (2006.01)

(52) Cooperative Patent Classification (CPC):
(C-Sets available)
**A61P 31/14; A61K 31/702; A61K 31/716; A61K 31/718; A61K 31/732; A61K 31/733; A61K 35/744; A61K 35/747; A61P 1/00** (Cont.)

(86) International application number:
**PCT/SE2021/051138**

(87) International publication number:
**WO 2022/103321 (19.05.2022 Gazette 2022/20)**

(54) **PROBIOTIC SYNBIONTIC COMPOSITION FOR USE IN THE TREATMENT OF INFLAMMATORY BOWEL SYNDROME (IBS)**

PROBIOTISCHE SYNBIOTISCHE ZUSAMMENSETZUNG ZUR VERWENDUNG BEI DER BEHANDLUNG DES ENTZÜNDLICHEN DARMSYNDROMS (IBS)

COMPOSITION SYNBIOTIQUE PROBIOTIQUE POUR UTILISATION DANS LE TRAITEMENT DU SYNDROME INFLAMMATOIRE DE L'INTESTIN (IBS)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.11.2020 SE 2051327**

(43) Date of publication of application:
**20.09.2023 Bulletin 2023/38**

(73) Proprietor: **Synbiotics AB**
**234 40 Lomma (SE)**

(72) Inventors:
- **SVENSSON, Ulla K**
  **223 61 LUND (SE)**
- **LAVEBRATT, Catharina**
  **192 73 SOLLENTUNA (SE)**
- **RÜEGG, Joëlle**
  **111 27 STOCKHOLM (SE)**
- **SCHALLING, Martin**
  **112 41 STOCKHOLM (SE)**

(74) Representative: **Ström & Gulliksson AB**
**P.O. Box 793**
**220 07 Lund (SE)**

(56) References cited:
**WO-A1-2005/077391 US-A1- 2007 286 916**
**US-A1- 2008 107 634 US-A1- 2012 114 608**
**US-A1- 2017 165 303 US-B2- 9 579 353**

- **KNIGHT DAVID J W ET AL: "Effect of synbiotic therapy on the incidence of ventilator associated pneumonia in critically ill patients: a randomised, double-blind, placebo-controlled trial", INTENSIVE CARE MEDICINE, SPRINGER BERLIN HEIDELBERG, BERLIN/HEIDELBERG, vol. 35, no. 5, 16 December 2008 (2008-12-16), XP037119694, ISSN: 0342-4642, [retrieved on 20081216], DOI: 10.1007/S00134-008-1368-1**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **KOTZAMPASSI KATERINA ET AL: "Benefits of a Synbiotic Formula (Synbiotic 2000Forte) in Critically Ill Trauma Patients: Early Results of a Randomized Controlled Trial", WORLD JOURNAL OF SURGERY, SPRINGER NEW YORK LLC, US, vol. 30, no. 10, 7 September 2006 (2006-09-07), pages 1848 - 1855, XP037139043, ISSN: 0364-2313, [retrieved on 20060907], DOI: 10.1007/S00268-005-0653-1**
- **BAUD DAVID ET AL: "Using Probiotics to Flatten the Curve of Coronavirus Disease COVID-2019 Pandemic", FRONTIERS IN PUBLIC HEALTH, vol. 8, 8 May 2020 (2020-05-08), XP055808426, DOI: 10.3389/fpubh.2020.00186**
- **SUNDARARAMAN ARAVIND ET AL: "Role of probiotics to combat viral infections with emphasis on COVID-19", APPLIED MICROBIOLOGY AND BIOTECHNOLOGY, SPRINGER BERLIN HEIDELBERG, BERLIN/ HEIDELBERG, vol. 104, no. 19, 19 August 2020 (2020-08-19), pages 8089 - 8104, XP037235769, ISSN: 0175-7598, [retrieved on 20200819], DOI: 10.1007/S00253-020-10832-4**



(52) Cooperative Patent Classification (CPC): (Cont.)

C-Sets
**A61K 31/702, A61K 2300/00;**
**A61K 31/716, A61K 2300/00;**
**A61K 31/718, A61K 2300/00;**
**A61K 31/732, A61K 2300/00;**
**A61K 31/733, A61K 2300/00;**
**A61K 35/744, A61K 2300/00;**
**A61K 35/747, A61K 2300/00**

## Description

### Technical Field

The present disclosure relates to a synbiotic composition, comprising probiotic bacterial strains and prebiotic fibres or oligosaccharides, for use in the treatment, prevention, amelioration, treatment, or reductions of symptoms of Inflammatory Bowel Disease (IBD) in an individual.

### Background art

During the last decades, the importance of the gut microbiota for wellbeing and health has been intensively studied. New knowledge has resulted in a suggestion to view the gut microbiota as a new organ. Molecular genetic methods have revolutionized the insight into the composition of the microbiota and microbiota analysis has made it possible to include studies of the microbiota as part of many human intervention studies. The gut with its microbiota has a key function to maintain immune homeostasis and approximately 70% of the immune system is estimated to be located in the gut.

Inflammatory bowel disease (IBD) is a term for two conditions (Crohn's disease and ulcerative colitis) that are characterized by chronic inflammation of the gastrointestinal (GI) tract. Prolonged inflammation results in damage to the GI tract. Some common symptoms are persistent diarrhea, abdominal pain, rectal bleeding/bloody stools, weight loss, and fatigue.

WO 2005/077391 A1 relates to the use of at least two lactic acid bacterial strains for the manufacturing of a formulation for the prevention or treatment of stress-induced inflammatory disorder.

US 9,579,353 B2 discloses a composition containing at least one probiotic microorganism strain comprising *Pediococcus* for ameliorating or reducing the symptoms, signs, and markers and for the treatment of irritable bowel syndrome, inflammatory bowel disease or gastritis,

An object of the present invention is to provide a composition for use in the treatment, prevention, amelioration, or reductions of symptoms of Inflammatory Bowel Disease (IBD) in an individual.

### Summary

According to a first aspect, the above and other objects of the invention are achieved, in full or at least in part by a composition as defined by claim 1. According to this claim, the above object is achieved by a synbiotic composition for use in the treatment, prevention, amelioration, or reductions of symptoms of Inflammatory Bowel Disease (IBD) in an individual, wherein the synbiotic composition comprises a *Lactobacillus paracasei* strain, wherein the *Lactobacillus paracasei* strain is *Lactobacillus paracasei* LMG P-17806 or *Lactobacillus paracasei* LMG P-26118; *Lactobacillus plantarum* LMG P-20606; *Bifidobacterium breve* LMG-P-26117; *Pediococcus pentosaceus* LMG P-20608; and *Leuconostoc mesenteroides* LMG P-20607; and at least one dietary fiber chosen from the group consisting of inulin, pectin, beta-glucan, resistant starch, a galacto-oligosaccharide, an isomalto-oligosaccharide, and a rice fiber.

According to one embodiment, the *Lactobacillus paracasei* strain is *Lactobacillus paracasei* (LMG P-17806.

According to one embodiment, the composition comprises inulin, pectin, beta-glucan and/or resistant starch.

According to another embodiment, the composition comprises resistant starch, a galacto-oligosaccharide, and/or an isomalto-oligosaccharide.

According to a further embodiment, the composition comprises resistant starch.

According to yet another embodiment, the composition further comprises inulin, pectin, and beta-glucan.

The composition may comprise a rice fiber.

The composition may comprise inulin, pectin, beta-glucan resistant starch and/or rice fibers.

According to one embodiment, the total amount of bacteria in one dose is $1\times10^{6}$ to $1\times10^{13}$, such as $5\times10^{6}$ to $1\times10^{13}$, such as $1\times10^{7}$ to $1\times10^{13}$, such as $5\times10^{7}$ to $1\times10^{13}$, such as $1\times10^{8}$ to $1\times10^{13}$, such as $5\times10^{8}$ to $5\times10^{12}$, especially $1\times10^{9}$ to $1\times10^{12}$, such as $5\times10^{9}$ to $9\times10^{11}$, such as $1\times10^{10}$ to $8\times10^{11}$, such as $5\times10^{10}$ to $7\times10^{11}$, such as $1\times10^{11}$ to $6\times10^{11}$, such as $3\times10^{11}$ to $5\times10^{11}$, such as $4\times10^{11}$ CFUs (colony forming units).

According to a further embodiment, the total amount of fibers in one dose is between 0.1 to 20 g, such as 0.5 to 20 g, such as 1 to 20 g, such as 2 to 15 g, such as 2.5 to 12.5 g, such as 5 to 10 g, such as 6 to 8.5 g, such as 7.5 g.

According to another embodiment, the Inflammatory Bowel Disease (IBD) is ulcerative colitis or Crohn's disease.

According to one embodiment, the synbiotic composition is administered orally or rectally or by tube feeding.

According to another embodiment, the synbiotic composition is administered as one dose 1 to 3 times a day, 1 to 7 times a week.

According to a further embodiment, the synbiotic composition is in the form of a powder, a capsule, a tablet, a lozenge, a liquid, an emulsion, an enema, a suppository, or a tube feeding.

According to another embodiment, the synbiotic composition is a food supplement, a food product, a nutritional supplement, a natural remedy or a pharmaceutical product.

The food product may be a biscuit, a cracker, a bar, or a liquid, e.g. a shot.

The bacterial strains disclosed here in have previously been deposited with the Belgian Coordinated Collections of Micro-organisms (BCCM), Laboratorium voor Microbiologie Bacteriënverzameling (LMG), Universiteit Gent, K.L. Ledenganckstraat 35, 9000 Gent, Belgium (Web site: http://bccm.belspo.be).

*Pediococcus pentosaceus* (LMG P-20608), *Lactobacillus plantarum* (LMG P-20606), and *Leuconostoc mesenteroides* (LMG P-20607) were deposited on June 19, 2001.

*Lactobacillus paracasei* (LMG P-17806) has been deposited with the Belgian Coordinated Collection of Microorganisms.

*Lactobacillus paracasei* (LMG P-26118) and *Bifidobacterium breve* (LMG P-26117) were deposited on November 24, 2010.

*Pediococcus pentosaceus* (LMG P-20608), *Lactobacillus plantarum* (LMG P-20606), *Lactobacillus paracasei* (LMG P-17806) and *Leuconostoc mesenteroides* (LMG P-20607) have previously been published in EP 1 624 762 B1.

*Lactobacillus paracasei* (LMG P-26118) and *Bifidobacterium breve* (LMG P-26117) have previously been published in EP 2 672 980 B1.

Other objectives, features and advantages of the present disclosure will appear from the following detailed description, from the experimental data, as well as from the attached claims. It is noted that the disclosure relates to all possible combinations of features.

Generally, all terms used in the claims are to be interpreted according to their ordinary meaning in the technical field, unless explicitly defined otherwise herein. All references to "a/an/the [component, means, step, etc.]" are to be interpreted openly as referring to at least one instance of said component, means, step, etc., unless explicitly stated otherwise. The steps of any method disclosed herein do not have to be performed in the exact order disclosed, unless explicitly stated.

As used herein, the term "comprising" and variations of that term are not intended to exclude other additives, components, integers or steps.

## Brief description of drawings

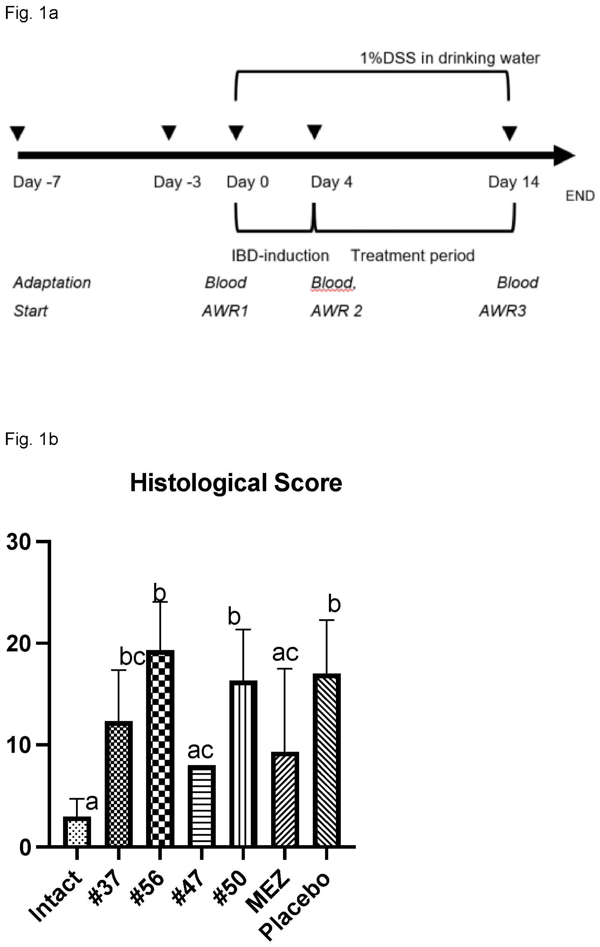

Fig. 1a shows a schematic overview of a study timetable, its design and animals' randomization.

Fig. 1b. shows histological evaluation of colon structure and colitis severity. Intact = Control group of intact healthy mice (n=3); #37, #56, #47, #50: animals receiving 1% DSS in drinking water and treated daily with respective products (Products 1-4) (n=3); MEZ: animals receiving 1% DSS in drinking water and treated daily with Mesalamine suspension in olive oil (100 mg/kg body weight) (n=3); Placebo: group receiving 1% DSS in drinking water and daily treated with water gavage as a vehicle (n=3). Data were analysed using one-way ANOVA test corrected for multiple comparisons and are presented as mean $\pm$ SD. Small letters describes significant differences when $p<0.05$.

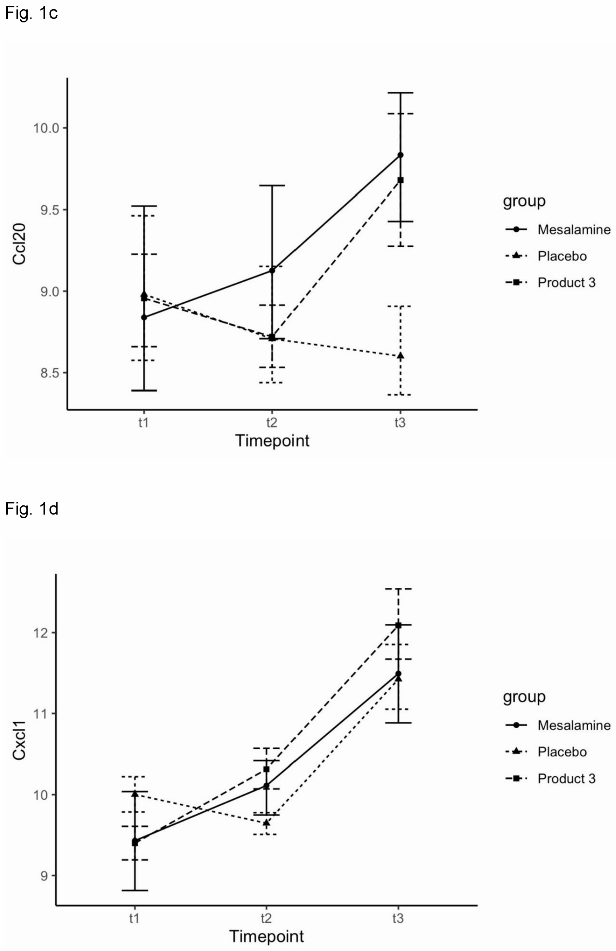

Figs. 1c-1f show the change in level over time for the 4 markers with statistically significant changes over time for all 4 products, here exemplified by the group treated with Product 3 (corresponding to Composition No. 47). THe marker changes in the group treated with clinical pharmacotherapy for IBD, Mesalamine, is also shown.

## Detailed description

The gut with its microbiota has a key function to maintain immune homeostasis and approximately 70% of the immune system is estimated to be located in the gut.

Probiotics are defined as "a live microorganisms which, when administered in adequate amounts, confer a health benefit on the host" (FAO/WHO 2002).

Fibres are an essential part of our diet. Fibres can be divided into different groups and the group called soluble fibres is important for the gut microbiota. A group of the soluble fibres and oligosaccharides are called prebiotics.

Prebiotics are defined as" a substrate that is selectively utilized by host microorganisms conferring a health benefit" (International Scientific Association for Probiotics and Prebiotics 2017). When prebiotics are fermented in the gut, short chain fatty acids can be produced. Examples of the short chain fatty acids are acetic acid, butyric acid, propionic acid, and formic acid. It is known that short chain fay acids influence health, e.g. butyric acid nourishes the gut mucosa and propionic acid influences fat metabolism.

Synbiotics is defined as "an ingredient or dietary supplement combining probiotics and prebiotics for a synergy between the compounds".

The combination of strains presented herein and used in the studies and experiments were carefully selected to

be optimal combination of strains for efficacy including bacterial survival and favourable influence on each other.

Different combinations of bacterial strains and fibers may be used in a composition according to the present disclosure. Different combinations of bacterial strains and different combinations of fibers are described below. Specific combinations of bacterial strains and fibers are shown in Table 1. However, the following is not to be interpreted as excluding other combinations of bacterial strains and fibers.

Throughout the present disclosure, specific bacterial strains are identified by their deposition number. Thus, *"Lactobacillus plantarum* (LMG P-20606)" can be denoted as *"Lactobacillus plantarum* (deposit no: LMG P-20606)" or *"Lactobacillus plantarum* LMG P-20606", all meaning a strain of *Lactobacillus plantarum* having the deposition number "LMG P-20606".

Compositions according to the present disclosure comprise a *Lactobacillus paracasei* strain, wherein the *Lactobacillus paracasei* strain is *Lactobacillus paracasei* LMG P-17806 or *Lactobacillus paracasei* LMG P-26118; *Lactobacillus plantarum* LMG P-20606; *Bifidobacterium breve* LMG-P-26117; *Pediococcus pentosaceus* LMG P-20608; and *Leuconostoc mesenteroides* LMG P-20607.

The *Lactobacillus paracasei* strain may be *Lactobacillus paracasei* (LMG P-17806).

The *Lactobacillus paracasei* strain may be *Lactobacillus paracasei* (*LMG P-26118*).

The composition may comprise *Lactobacillus plantarum* (LMG P-20606); *Lactobacillus paracasei* (LMG P-17806); *Pediococcus pentosacceus* (LMG P-20608); *Leuconostoc mesenterioides* (LMG P-20607); and *Bifidobacterium breve* (LMG P-26117).

The composition may comprise *Lactobacillus plantarum* (LMG P-20606); *Lactobacillus paracasei* (LMG P-26118); *Pediococcus pentosacceus* (LMG P-20608); *Leuconostoc mesenterioides* (LMG P-20607); and *Bifidobacterium breve* (LMG P-26117).

Compositions according to the present disclosure comprise at least one fiber chosen from the group consisting of inulin, pectin, beta-glucan, resistant starch, a galacto-oligosaccharide, an isomalto-oligosaccharide, and a rice fiber.

The composition may comprise resistant starch.

The composition may comprise inulin, pectin, beta-glucan and resistant starch.

The composition may comprise inulin, pectin, beta-glucan, rice fiber and resistant starch.

The composition may comprise resistant starch and a galacto-oligosaccharide.

The composition may comprise resistant starch and an isomalto-oligosaccharide.

The composition may comprise resistant starch, galacto-oligosaccharide and isomalto-oligosaccharide.

A composition according to the present invention may be administered one to three times a day.

Some examples of compositions according to the present invention as well as compositions outside of the scope of the claims (Compositions 1-36 and 43-57) are shown in Table 1 below.

A composition according to the present invention may be administered one to seven days a week.

Preferably, a composition according to the present invention is administered once a day as a single dose.

The total amount of bacteria in one dose of a composition according to the present disclosure is between $1\times10^6$ to $1\times10^{13}$, such as $5\times10^6$ to $1\times10^{13}$, such as $1\times10^7$ to $1\times10^{13}$, such as $5\times10^7$ to $1\times10^{13}$, such as $1\times10^8$ to $1\times10^{13}$, such as $5\times10^8$ to $5\times10^{12}$, especially $1\times10^9$ to $1\times10^{12}$, such as $5\times10^9$ to $9\times10^{11}$, such as $1\times10^{10}$ to $8\times10^{11}$, such as $5\times10^{10}$ to $7\times10^{11}$, such as $1\times10^{11}$ to $6\times10^{11}$, such as $3\times10^{11}$ to $5\times10^{11}$, such as $4\times10^{11}$ CFUs (colony forming units).

The total amount of fibers in one dose of a composition according to the present disclosure is between 0.1 to 20 g, such as 0.5 to 20 g, such as 1 to 20 g, such as 2 to 15 g, such as 2.5 to 12.5 g, such as 5 to 10 g, such as 6 to 8.5 g, such as 7.5 g.

A composition according to the present disclosure may be in the form of a dry powder intended to be mixed with a liquid, preferably water, before intake.

A composition according to the present disclosure may be in the form of a capsule, a tablet, a lozenge, a liquid, an emulsion, an enema, a suppository, or a tube feeding.

***Table 1.*** *Compositions according to the present invention as well as compositions outside the scope of the claims (Compositions 1-36 and 43-57). The total amount of bacteria in one dose is between* $1\times10^{6}$ to $1\times10^{13}$, such as $5\times10^{6}$ to $1\times10^{13}$, such as $1\times10^{7}$ to $1\times10^{13}$, such as $5\times10^{7}$ to $1\times10^{13}$, such as *$1\times10^{8}$ to $1\times10^{13}$, such as $5\times10^{8}$ to $5\times10^{12}$, especially $1\times10^{9}$ to $1\times10^{12}$, such as $5\times10^{9}$ to $9\times10^{11}$, such as $1\times10^{10}$ to $8\times10^{11}$, such as $5\times10^{10}$ to $7\times10^{11}$, such as $1\times10^{11}$ to $6\times10^{11}$, such as $3\times10^{11}$ to $5\times10^{11}$, such as $4\times10^{11}$ CFUs (colony forming units). The total amount of fibers in one dose is between 0.1 to 20 g, such as 0.5 to 20 g, such as 1 to 20 g, such as 2 to 15 g, such as 2.5 to 12.5 g, such as 5 to 10 g, such as 6 to 8.5 g, such as 7.5 g. In addition to the fibers listed below, rice fiber may also be present. Combinations No. 1-57 also including rice fibers are thus also possible.*

| **Composition No.** | **Bacterial strains (at least two)** | | | | | | **Fiber (at least one)** | | | | | |
|---|---|---|---|---|---|---|---|---|---|---|---|---|
| | *L. plantarum* (LMG P-20606) | *L. paracasei* (LMG P-17806) | *L. paracasei* (LMG P-26118) | *P. pentosaceus* (LMG P-20608) | *L. mesenteroides* (LMG P-20607) | *B. breve* (LMG-P-26117) | inulin | pectin | beta-glucan | resistant starch | galacto-oligosaccharide | isomalto-oligosaccharide |
| **1** | x | x | | x | | | x | x | x | x | | |
| **2** | x | x | | x | | | | | | x | | |
| **3** | x | x | | x | | | | | | x | x | x |
| **4** | x | | x | x | | | x | x | x | x | | |
| **5** | x | | x | x | | | | | | x | | |
| **6** | x | | x | x | | | | | | x | x | x |
| **7** | x | x | | | x | | x | x | x | x | | |
| **8** | x | x | | | x | | | | | x | | |
| **9** | x | x | | | x | | | | | x | x | x |
| **10** | x | | x | | x | | x | x | x | x | | |

| | | | | | | | | | | | | |
|---|---|---|---|---|---|---|---|---|---|---|---|---|
| **11** | x | | x | | x | | | | | x | | |
| **12** | x | | x | | x | | | | | x | x | x |
| **13** | x | x | | | | x | x | x | x | x | | |
| **14** | x | x | | | | x | | | | x | | |
| **15** | x | x | | | | x | | | | x | x | x |
| **16** | x | | x | | | x | x | x | x | x | | |
| **17** | x | | x | | | x | | | | x | | |
| **18** | x | | x | | | x | | | | x | x | x |
| **19** | x | x | | x | x | | x | x | x | x | | |
| **20** | x | x | | x | x | | | | | x | | |
| **21** | x | x | | x | x | | | | | x | x | x |
| **22** | x | | x | x | x | | x | x | x | x | | |
| **23** | x | | x | x | x | | | | | x | | |
| **24** | x | | x | x | x | | | | | x | x | x |
| **25** | x | x | | x | | x | x | x | x | x | | |
| **26** | x | x | | x | | x | | | | x | | |
| **27** | x | x | | x | | x | | | | x | x | x |
| **28** | x | | x | x | | x | x | x | x | x | | |
| **29** | x | | x | x | | x | | | | x | | |
| **30** | x | | x | x | | x | | | | x | x | x |
| **31** | x | x | | | x | x | x | x | x | x | | |
| **32** | x | x | | | x | x | | | | x | | |
| **33** | x | x | | | x | x | | | | x | x | x |
| **34** | x | | x | | x | x | x | x | x | x | | |
| **35** | x | | x | | x | x | | | | x | | |
| **36** | x | | x | | x | x | | | | x | x | x |
| **37** | x | x | | x | x | x | x | x | x | x | | |
| **38** | x | x | | x | x | x | | | | x | | |
| **39** | x | x | | x | x | x | | | | x | x | x |
| **40** | x | | x | x | x | x | x | x | x | x | | |
| **41** | x | | x | x | x | x | | | | x | | |
| **42** | x | | x | x | x | x | | | | x | x | x |

| | | | | | | | | | | | | |
|---|---|---|---|---|---|---|---|---|---|---|---|---|
| **43** | X | X | | | | | X | X | X | X | | |
| **44** | X | X | | | | | | | | X | | |
| **45** | X | X | | | | | | | | X | X | X |
| **46** | X | | X | | | | X | X | X | X | | |
| **47** | X | | X | | | | | | | X | | |
| **48** | X | | X | | | | | | | X | X | X |
| **49** | X | | | X | | | X | X | X | X | | |
| **50** | X | | | X | | | | | | X | | |
| **51** | X | | | X | | | | | | X | X | X |
| **52** | X | | | | X | | X | X | X | X | | |
| **53** | X | | | | X | | | | | X | | |
| **54** | X | | | | X | | | | | X | X | X |
| **55** | X | | | | | X | X | X | X | X | | |
| **56** | X | | | | | X | | | | X | | |
| **57** | X | | | | | X | | | | X | X | X |

Inflammatory bowel disease (IBD) is a term for two conditions (Crohn's disease and ulcerative colitis) that are characterized by chronic inflammation of the gastrointestinal (GI) tract. Prolonged inflammation results in damage to the GI tract. Some common symptoms are persistent diarrhea, abdominal pain, rectal bleeding/bloody stools, weight loss, and fatigue. One or more of these symptoms may be treated, ameliorated or reduced according to the present disclosure.

One composition that is specifically suitable for the treatment, prevention, amelioration or reduction of symptoms of IBD in an individual comprises *Lactobacillus plantarum* (LMG P-20606); *Lactobacillus paracasei* (LMG P-17806); *Pediococcus pentosacceus* (LMG P-20608); *Leuconostoc mesenterioides* (LMG P-20607), and *Bifidobacterium breve* (LMG P-26117).

Another composition that is specifically suitable for the treatment, preventon, amelioration or reduction of symptoms of IBD in an individual comprises *Lactobacillus plantarum* (LMG P-20606); *Lactobacillus paracasei* (LMG P-26118); *Pediococcus pentosacceus* (LMG P-20608); *Leuconostoc mesenterioides* (LMG P-20607); and *Bifidobacterium breve* (LMG P-26117).

Compositions that are specifically suitable for the treatment, amelioration or reduction of symptoms of IBD are Composition Nos. 37 and 40 in Table 1.

Examples of areas where the microbiota has been shown to play an important role is on various immune functions including inflammation and on the gut-brain axis. The gut-brain axis is the bidirectional interaction between the gut and the central nervus system. Molecules of importance for this interaction are e.g. short chain fatty acids (SCFAs) which can be produced by the gut microbiota if a suitable substrate is available e.g. soluble fibres or oligosaccharides. The most abundant short chain fatty acids (SCFAs) in stool and body fluids are formic acid, acetic acid, propionic acid and butyric acid. SCFAs are multifunctional molecules, being e.g. an essential energy source for local intestinal cells, but also influencing barrier function, neurotransmitter release, microglial maturation and activation, neural proliferation, mitochondrial function, oxidative stress, immune-modulation, and anti-inflammatory processes. SCFAs can regulate brain function and behavior via a number of potential mechanisms and dysregulation of these pathways may contribute to autism and related disorders. Butyric acid, formic acid, acetic acid and propionic acid have previously been reported to have anti-inflammatory effects. SCFAs regulate several leukocyte functions including production of cytokines eicosanoids and chemokines. The ability of leukocytes to migrate to the foci of inflammation and to destroy microbial pathogens also seems to be affected by the SCFAs. Studies have shown that SCFA at physiological concentration may affect the pathogenesis of some viruses e.g. Herpesvirus at the epithelial site.

VCAM-1 (Vascular Cell Adhesion Molecule) is a receptor used by some types of Adenovirus during infection. VCAM-1 is a cell surface glycoprotein expressed on e.g. endothelial cells and immune cells such as macrophages and

dendritic cells. VCAM-1 was originally identified as a cell adhesion molecule that helps to regulate inflammation-associated vascular adhesion and the transendothelial migration of leukocytes, such as macrophages and T cells. Recent evidence suggests that VCAM-1 is closely associated with the progression of various immunological disorders.

ICAM-1 (InterCellular Adhesion Molecule) is a receptor used by most Rhinoviruses and some Coxsackievirus as a receptor during infection. ICAM-1 is also important for the infection cycle of influenzavirus. ICAM-1 (also known as CD54 (Cluster of Difference 54)) is a cell surface glycoprotein that promotes signalling in between cells related to the immune response. ICAM-1 is expressed on endothelial cells, immune cells and on respiratory endothelium where it can serve as a receptor for viruses, e.g. human rhinovirus (HRV).

IL-12/IL23p40 has been identified as important in Inflammatory Bowel Disease (IBD), i.e. in ulcerative colitis and Crohn's disease, and is a target for medical treatment of the disease. Thus, reducing the expression of IL-12/IL-23p40 is anticipated to have an effect on the clinical manifestation of this disease.

In the experiments described below, the effect of a composition (corresponding to Composition No. 1) on the levels of i.a. IL-12/IL-23p40, ICAM-1, and SCFAs was studied.

## Experiments and studies

### *Immunological markers and short chain fatty acids in healthy individuals and individuals with inflammation*

The levels of selected immunological markers and short chain fatty acids were measured in blood samples from healthy individuals (n=57) and individuals diagnosed with inflammatory disorder/inflammatory load/inflammatory burden (n=154, 8-55 years old, both men and women). A variety of immune activity markers of which some also are virus receptors were analysed.

As can be seen in Table 2, individuals diagnosed with inflammatory disorder/inflammatory load/inflammatory burden have significantly ($p < 0.005$) lower expression of IL-10 (an anti-inflammatory marker) and significantly ($p < 0.005$) higher expression of the inflammatory markers and upper tract respiratory disease virus receptors sICAM-1and sVCAM-1.

This clearly demonstrates that individuals with inflammatory disorder/inflammatory load/inflammatory burden express an inflammatory pattern of immune markers and express virus receptors at higher levels than healthy individuals.

***Table 2.*** *Inflammatory activity markers/virus receptors, expressed in healthy individuals ("Control group") and individuals diagnosed with inflammatory disorder/linflammatory load/inflammatory burden ("Inflammatory group")*

| Marker/ receptor | Control group Amount pg/ml (median) | Inflammatory group at baseline Amount pg/ml (median) | Significance, difference between control group and inflammatory group (p) |
|---|---|---|---|
| **IL-10** | 0.60 | 0.25 | 0.00202 |
| **sICAM-1** | 400,000 | 500,000 | 0.00202 |
| **sVCAM-1** | 400,000 | 480,000 | 0.00003 |

Formic and propionic acids (short chain fatty acids) were lower in the individuals with inflammatory disorder/inflammatory load/inflammatory burden compared to the control group of 57 healthy individuals. Short chain fatty acids in plasma correlated positively with IL-10, i.e. individuals with a low level of short chain fatty acids also had a low level of the anti-inflammatory cytokine IL-10. This was seen both in children and in adults (data not shown).

### *Influence of a synbiotic composition corresponding to Composition No. 1 (not within the scope of the claims) on inflammatory markers and virus receptors in individuals with inflammation*

A human intervention study was performed in a group of individuals (n=49, children, both boys and girls) diagnosed with inflammatory disorder/inflammatory load/inflammatory burden. The effect of a synbiotic composition corresponding to Composition No. 1 on plasma levels of immune markers, virus receptors and short fatty acids in children diagnosed with inflammatory disorder/inflammatory load/inflammatory burden was investigated. The synbiotic composition included *Lactobacillus plantarum* LMG P-20606, *Lactobacillus paracasei* LMG P-17806, *Pediococcus pentosaceus* LMG P-20608 and the fibres inulin, pectin, resistant starch and beta-glucan. The total amount of bacteria was $4\times10^{11}$ and the total amount of fibres was 10g. The individuals diagnosed with inflammatory disorder/inflammatory load/inflammatory burden were randomized into one group (n=28) receiving the synbiotic composition and one group (n=21) receiving a maltodextrin placebo. The synbiotic composition was consumed once daily for nine weeks. Immune markers and virus receptors were analysed in plasma at start of the study (T=0) and after nine weeks of intervention (T=9).

After 9 weeks, children receiving the synbiotic composition had a significantly ($p<0.05$) reduced level of the

inflammatory marker IL-12/IL-23p40, the upper tract respiratory disease virus receptor sICAM and of transforming growth factor β3 (TGF-β3) in plasma compared to the levels at start of the intervention study (Table 3).

The results clearly show that the synbiotic composition lowered the expression of IL-12/IL-23p40, sICAM and TGF-β3, which all are inflammatory marker. IL-12/IL-23p40 is a target for drugs against inflammatory diseases and ICAM also serves as a receptor for viruses. Thus, intake of the synbiotic composition lowers the level of an important receptor for viruses infecting the upper respiratory tract, thus reducing the entry points of these viruses. Furthermore, the results show that the synbiotic composition lowers the expression of the pro-inflammatory molecules IL-12/IL-23p40, a target, the reduction of which is used in the treatment of ulcerative colitis and/or Crohn's disease. Thus, a synbiotic composition including *Lactobacillus plantarum* LMG P-20606, *Lactobacillus paracasei* LMG P-17806, *Pediococcus pentosaceus* LMG P-20608 and the fibres inulin, pectin, resistant starch and beta-glucan can be used in the alleviation or treatment of ulcerative colitis and/or Crohn's disease.

***Table 3.*** *Time effects over 9 weeks on inflammatory markers*

| Marker/receptor | T=0 Amount pg/ml (median) | T=9 weeks Amount pg/ml (median) | Significance difference between T=0 and T=9 weeks (p) |
|---|---|---|---|
| **IL-12/IL-23p40** | 260 | 238 | 0.0281 |
| **sICAM-1** | 740,000 | 670,000 | 0.0242 |
| **TGF-β3** | 4.1 | 3.6 | 0.0405 |

***Influence of a synbiotic composition on short chain fatty acids and their correlation to inflammatory markers and virus receptors***

A human intervention study was performed in a group of individuals (n=49, children, both boys and girls) diagnosed with inflammatory disorder/inflammatory load/inflammatory burden as described above. The synbiotic composition increased the levels of propionic and acetic acid, which correlated negatively to the levels of sICAM-1 and sVCAM-1, i.e. the proinflammatory markers and receptors were high when the short chain fatty acid level was low (data not shown). Thus, treatment with the synbiotic composition is associated with lower vascular inflammation. The results clearly show that the level of short chain fatty acids is associated to the level of immune markers and receptors, and with low levels of short chain fatty acids associated to a pro-inflammatory pattern of the immune marker and an enhanced expression of the virus receptors. In the study it was also clear that the synbiotic composition increased the plasma level of short chain fatty acids which will stimulate a low inflammation.

***In vivo* IBD-study**

*Objective and method*

A study was conducted in mice with the objective to demonstrate the effect of test products (different pre-and probiotics compositions) on intestinal inflammation induced by dextran sodium sulfate (DSS).

This study followed principles of Good Laboratory Practice (GLP) as defined by the Organization for Economic Co-operation and Development (OECD) as applicable. Procedures involving the care and the use of animals in the study was reviewed by the University of Lund Ethics Review Committee on Animal Experiments. During the study, the care and use of animals was conducted in accordance with the principles outlined in the current Guide to the Care and Use of Experimental Animals.

The study was conducted by the Dept Biology, Lund University.

*Tested products*

Tested products were a combination of probiotic bacteria and prebiotic fibres:

Product 1 (corresponding to Composition No. 37 in Table 1; also referred to as SB1): *Lactobacillus plantarum* (LMG P-20606); *Lactobacillus paracasei* (LMG P-17806); *Pediococcus pentosacceus* (LMG P-20608); *Leuconostoc mesenterioides* (LMG P-20607); *Bifidobacterium breve* (LMG P-26117); β-glucan from oat; Inulin from chicory root; Pectin from citrus fruits; and Resistant starch from corn.

Product 2 (corresponding to Composition No. 56 (not within the scope of the claims) in Table 1; also referred to as SB2): *Lactobacillus plantarum* (LMG P-20606); *Bifidobacterium breve* (LMG P-26117); and Resistant starch from corn.

Product 3 (corresponding to Composition No. 47 (not within the scope of the claims) in Table 1; also referred to as SB3): *Lactobacillus plantarum* (LMG P-20606); *Lactobacillus paracasei* (LMG P-26118); and Resistant starch from corn.

Product 4 (corresponding to Composition No. 50 (not within the scope of the claims) in Table 1; also referred to as SB4): *Lactobacillus plantarum* (LMG P-20606); *Pediococcus pentosacceus* (LMG P-20608); and Resistant starch from corn.

*Control groups*

Three control groups were run in parallel, one which was only run in parallel to the experiment but where the mice were not treated with anything, one given only 1% DSS in drinking water and no pro/prebiotics and one that was given 1% DSS and conventional treatment with mesalamine.

*Animals used in the investigation:*

In all the groups, the ones given probiotics (i.e. Product 1, 2, 3 and 4 corresponding to composition 37, 56, 47 or 50) and the three control groups, twelve mice were included in each group. For initial testing of colon sensitivity, two groups of mice, one given no treatment and one given 1% DSS for 4 days, were used before gut sensitivity testing.

The daily dose of bacteria to a mouse was 2x10E8 CFU. The daily dose of fiber to a mouse was 0.0025 g /day.

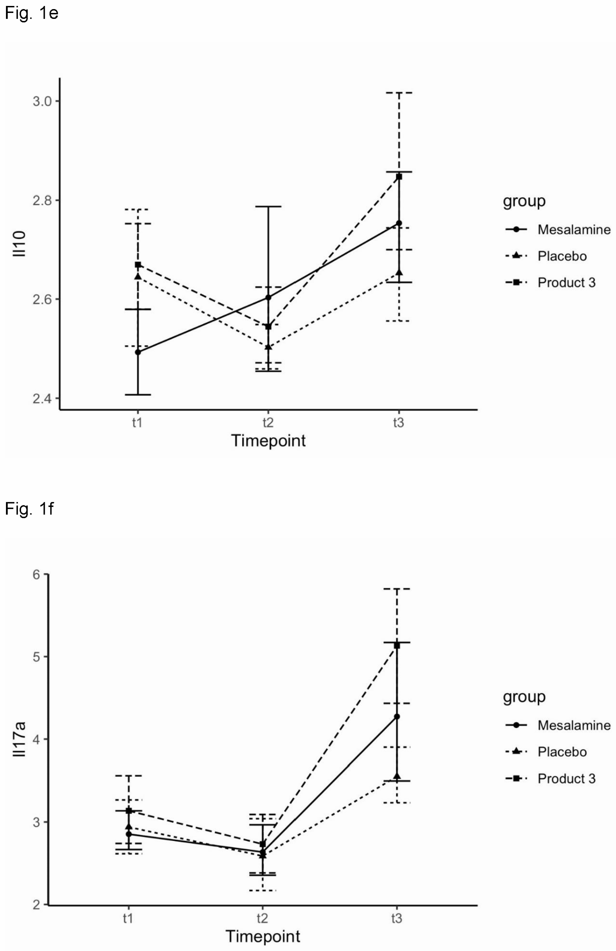

Female and male mice of the line C57BL were used. The age of the animals was 10 weeks. Average weight of the animals was 20-25 g at the start. The mice were fed a standard commercial diet (Lactamin, Vadstena, Sweden) which was available ad libitum as was water. Animal body weight and feed intake after adaptation period was measured daily, and water intake measured weekly. All animals but the control group, without any treatment, was given 1% DSS to induce an inflammation in the gut. A schematic overview of the study timetable, its design and animals' randomization is given in Fig. 1.

*Sampling*

Blood samples were collected from the submandibular vein and isolated plasma was frozen at -80°C until analysis (plasma immunological profile).

Stool samples were collected both before starting the treatment as well as after DSS treatment and after product treatment. Stool samples were frozen until analysis of calprotectin using S100A8/A9 (MRP8/14, Calprotectin, Mouse/Rat) ELISA kit, KR6936, ImmunDiagnostik, Bensheim, Germany).

The study was finalized by all animals euthanized at the end of the study by treatment by an overdose of isoflurane/transcardial perfusion.

For post-mortem analysis and tissue sampling, half of the mice from each group was euthanized by isoflurane overdose. Spleen and colon were dissected from these mice and weighed and colon length was measured. Samples of large intestine (caecum, proximal and distal colon) were collected and fixed in 10% neutral formalin solution fixative for 24 hours for further morphological analysis.

The other half of the mice from each group was euthanized by transcardial perfusion with Thyrodes solution + LANA's Fixative. Caecum, proximal and distal colon was dissected from these mice. The intestinal samples were kept in the same fixative overnight and then transferred to 70% alcohol for further morphological analysis.

*Analysis*

Mice colon hypersensitivity was assessed by measuring the threshold intensity of the abdominal withdrawal reflex (AWR) arising in response to colorectal distention. Distention was applied using an arterial embolectomy catheter (4F-Fogarty, Edwards Lifesciences LLC, Irvine, CA, USA) which was inserted rectally into the descending colon of anesthetized mice (2% isoflurane) and fixed at the base of the tail. AWR measurements were carried out 30 min after wake-up and reorientation of the animals. A visual observation of the reaction to the rapid phase stretching of the balloon for 20 s in ascending order (0.1, 0.25, 0.35, 0.5, and 0.65 ml) was carried out. The response of the animal to colorectal distention was assessed on an AWR scale: 0, no behavioral response; 1, brief movement of the head, followed by immobility; 2, contraction of abdominal muscles; 3, lifting the abdomen, and 4, body flexion and pelvic lift. The visceral pain threshold (VPT) was defined by the stimulus intensity whereby to evoke a visible contraction of the abdominal wall.

Morphological analysis was performed for three intestinal segments for six animals/group.

At dissection, samples were collected from the large intestine: caecum, proximal and distal colon, and fixed in 10% neutral formalin solution fixative for 24 hours. Next, tissue pieces were dehydrated and embedded in paraffin according to standard histological techniques. Paraffin-embedded tissues were sliced using rotor microtome into sections

4.5 microns (μm) thick. After overnight drying, slides were deparaffinized by incubation in xylene and decreasing concentrations of ethanol. Finally, standard hematoxylin and eosin staining was performed for morphology analysis.

Statistical analysis was performed as follows: The distribution of the parameters was checked using a Shapiro-Wilk normality test. One-way ANOVA or Kruskal-Wallis test corrected for multiple comparisons was used dependently on the data distribution. In all statistical analyses $p \leq 0.05$ was considered significant. All analyses were carried out using Prism, version 9.1.0 (GraphPad Software, Inc, San Diego, CA, USA).

*Analysis of biological markers in plasma*

Blood samples collected in EDTA tubes from the mice and plasma was collected. Selected biomarkers were analysed using Proximity Extension Assay (PEA) technology at Olink, Uppsala, Sweden. The technique combines immune technology with qPCR enabling a high-throughput, multiplex immunoassays of proteins using minimal volumes of serum, plasma, or almost any other type of biological sample. In total 75 different markers were analysed.

Plasma samples from all experimental groups were analysed with the PEA-technique.

*Results*

To indicate colitis development and its severity in mice, clinical assessment of animals was performed daily. Clinical scores indicated the colitis development already at day 3 after 1% DSS introduction. Neither Mesalamine, which is approved by FDA for the treatment for IBD, nor the tested Products could influence body weight reduction. However, administration of Products 1, 2, and 3 led to significant ($p<0,05$) improvement in animals' wellbeing and general health status, including better stool consistency and faecal blood disappearance (Table 4a). The clinical score of mice from these groups (Products 1, 2, and 3) on the last day of study (Day 14) was significantly ($p<0.05$) lower than in the Placebo group and significantly lower than in the Mesalamine group i.e. when the mice were treated by a conventional treatment for IBD. Moreover, clinical score values observed in SB1 (Product 1) treated animals didn't differ from the those noted for healthy intact mice from Control group.

In conclusion: The Products 1, 2 and 3 (corresponding to Compositions No. 37, 56 and 47) led to a significant, 50-68%, improvement in health status in mice where IBD had been induced by 1% DSS (Table 4a). The effect seen from those three synbiotic products was also significantly better than when the conventional treatment with Mesalamine was used. This clearly indicates that compositions according to the present disclosure can be used in the prevention, amelioration, treatment, or reductions of symptoms in an individual suffering from IBD.

***Table 4a.*** *Clinical score general health on Day 14.*

| Group | Body weight gain, % of initial body weight | Clinical Score |
|---|---|---|
| **Control** | 108.7±6.3a | 0.0±0.0a |
| **Product 1 (corresponding to Composition No. 37)** | 90.4±8.8b | 2.4±2.0ab |
| **Product 2 (corresponding to Composition No. 56)** | 90.0±9.5b | 3.5±3.0b |
| **Product 3 (corresponding to Composition No. 47)** | 90.2±10.5b | 3.6±2.9b |
| **Product 4 (corresponding to Composition No. 50)** | 86.2±10.0b | 6.0±3.8bc |
| **MEZ** | 88.0±9.3b | 4.8±2.1bc |
| **Placebo** | 90.8±12.5b | 7.4±1.2c |

*Control: group of intact healthy mice (n=12); Product 1-Product 4: animals receiving 1% DSS in drinking water and treated daily with respective products (Products 1-4) (n=11-12); MEZ: animals receiving 1% DSS in drinking water and treated daily with Mesalamine suspension in olive oil (100 mg/kg body weight) (n=11); Placebo: group receiving 1% DSS in drinking water and daily treated with water gavage as a vehicle (n=12). Data were analysed using one-way ANOVA test (body weight gain) and Kruskal-Wallis test (clinical score) corrected for multiple comparisons and are presented as mean ± SD. Small letters describes significant differences within columns when p<0.05.*

The well-established marker for IBD and gut inflammation calprotectin was analysed in faeces after 1% DSS treatment showing that already after 4 days of treatment there was a significant 3-5 fold increase in the marker (Table 4b). The conventional treatment by mesalamine did not reduce the effect of DSS, but treatment with Products 1 and 2 prevented further increment of calprotectin levels and Product 3 significantly reduced ($P<0.05$) fecal calprotectin by 49%.

In conclusion: Products 1 and 2 (corresponding to Compositions No. 37 and 56, respectively) prevented increment in calprotectin and Product 3 (corresponding to Composition No. 47) significantly reduced fecal calprotectin with 49%. This clearly indicates that compositions according to the present disclosure can be used in the prevention, amelioration, treatment, or reductions of symptoms in an individual suffering from IBD.

***Table 4b.*** *Changes in faecal calprotectin levels.*

| | **Calprotectin, %** | | |
|---|---|---|---|
| | **Day -3** | **Day 4** | **Day 14** |
| **Control** | 100.00±0.00a | 83.46±48.03a | 109.26±41.87a |
| **<u>Product 1</u> (corresponding to Composition No. 37** | 100.00±0.00a | 215.28±48.44b | 202.75±105.48ab |
| **<u>Product 2</u> (corresponding to Composition No. 56)** | 100.00±0.00a | 521.15±323.22b | 257.46±235.88ab |
| **<u>Product 3</u> (corresponding to Composition No. 47)** | 100.00±0.00a | 457.35±261.72b | 283.60±112.67c |
| **<u>Product 4</u> (corresponding to Composition No. 50)** | 100.00±0.00a | 260.97±112.67b | 273.55±189.38b |
| **MEZ** | 100.00±0.00a | 207.87±51.58b | 429.60±188.45c |
| **Placebo** | 100.00±0.00a | 294.62±137.95b | 377.54±234.38b |

*Control: group of intact healthy mice (n=12); Product 1-Product 4: animals receiving 1% DSS in drinking water and treated daily with respective products (Products 1-4) (n=11-12); MEZ: animals receiving 1% DSS in drinking water and treated daily with Mesalamine suspension in olive oil (100 mg/kg body weight) (n=11); Placebo: group receiving 1% DSS in drinking water and daily treated with water gavage as a vehicle (n=12). Data were analysed using one-way ANOVA test (body weight gain) and Kruskal-Wallis test (clinical score) corrected for multiple comparisons and are presented as mean ± SD. Small letters describes significant differences within columns when p<0.05.*

1% DSS-induced intestinal inflammation was characterized by inflammatory cell infiltration, abnormal crypt architecture, hyperplasia and edema. Some mild histological alterations were detected in the intact control group, however, all animals treated with DSS developed colitis. However, Mesalamine and Product 3 treatment significantly reduced inflammation, improved crypt structure and ameliorated edema by ca 50 % (Fig. 1b).

In conclusion: Product 3 (corresponding to Composition No. 47) treatment significantly reduced inflammation, improved crypt structure and ameliorated edema by ca 50 %. This clearly indicates that compositions according to the present disclosure can be used in the prevention, amelioration, treatment, or reductions of symptoms in an individual suffering from IBD.

The use of the synbiotic products significantly influenced several plasma biomarkers and in particular immunoactive markers. Three markers were influenced similarly independently of the product used. Figs 1c-1f show results for those markers, i.e. Ccl20, IL17a and Cxcl1, in plasma in the group of mice receiving product 3 (composition 47). The pattern of reaction is similar for these three markers when any of the four combination of the synbiotic composition was used i.e. the amount of the marker is relatively stable after DSS treatment (t=2) but increases significantly after treatment with the synbiotic composition (t=3), thus the figures are typical examples of what happens when the synbiotic products are given to mice after IBD induction. The increase in the amount of the markers is usually a little higher than the increase after mesalamine treatment. Mesalamine is an established way of treating IBD. For the anti-inflammatory cytokine IL10 the pattern is somewhat different i.e. the amount of this marker in plasma decreases significantly after treatment with DSS but increases again when the synbiotic product is given. This result on IL10 was only significant in the group receiving product 3. Other markers influenced by the different synbiotic compositions is different between the products meaning that the specific composition of a product is important for a specific effect (Table 4c).

***Table 4c.*** *Plasma markers where the change over time is different in Product compared to placebo, at T1 to T2, T2 to T3, and T1 to T3. Markers with p-value below 0.05 in T2 to T3 or T1 to T3 are listed.*

| | **Placebo Mean (SD)** | | | **Product Mean (5D)** | | | **p-value$_{Active}$ compared to Placebo** | | |
|---|---|---|---|---|---|---|---|---|---|
| **Marker** | **T2 minus T1** | **T3 minus T2** | **T3 minus T1** | **T2 minus T1** | **T3 minus T2** | **T3 minus T1** | **T2 minus T1** | **T3 minus T2** | **T3 minus T1** |
| ***Product 1*** | | | | | | | | | |
| Il17a | 0.3519(0.5937) | -0.9644(0.8592) | 0.6125(0.7647) | 0.139(0.5627) | -3,085(2,1354) | -2,946(1.9258) | 0.46 | 0.00032 | 0.0000914 |
| Tnfrsf11b | 0.1277(0.3085) | -0.0803(0.2906) | 0.0473(0.1606) | -0.05(0.2705} | -0.713(0.4571) | -0.763(0.3544) | 0.237 | 0.00306 | 0.0000457 |
| Ccl20 | 0.2731(0.549) | 0.1061(0.3342) | 0.3793(0.5802) | 0.317(0.7606) | -0.555(0.8594) | -0.238(1.1855) | 0.897 | 0.0205 | 0.0205 |
| Vegfd | -0.0589(0.2476) | 0.1792(0.2245) | 0.1203(0.2777) | 0.05(0.1368) | -0.032(0.1507) | 0.018(0.1601) | 0.173 | 0.0266 | 0.515 |
| Lpl | -0.504(0.3032) | -0.0582(0.3419) | 0.5623(0.5387) | -0.006(0.4629) | -0.428(0.3984) | -0.434(0.5149) | 0.0117 | 0.0676 | 0.696 |
| Tpp1 | 0.0955(0.2996) | 0.0681(0.3335) | 0.1636(0.3649) | -0.044(0.2442) | -0.336(0.4341) | -0.38{0.413) | 0.237 | 0.0676 | 0.00439 |
| Il17f | 0.093(0.4158) | -0.4155(0.6329) | 0.4186(0.7575) | -0.035(0.6452) | -1.473(1.5854) | -1.556(1.4008) | | 1 0.106 | 0.0426 |
| Ccl5 | -0.226(0.2751) | -0.0106(0.3212) | 0.2806(0.2763) | 0.191(0.2214) | 0.117(0.3518) | 0.363(0.2881) | 0.00967 | 0.272 | 0.00122 |
| Cxcl1 | 0.3554(0.3142) | -1.7778(0.5756) | 1.4225(0.7439) | -0.185(0.4764) | -2.477(0.9796) | -2.662(1.2783) | 0.0117 | 0.122 | 0.0266 |
| ***Product 2*** | | | | | | | | | |
| Prdx5 | -0.0496(0.4163) | -0.2095(0.4065) | 0.2591(0.5839) | -0.285(0.5765) | -1.786(1.4823) | -1.989(1.8422) | 0.44 | 0.0312 | 0.0727 |
| Il17a | 0.3519(0.5937) | -0.9644(0.8592) | 0.6125(0.7647) | 0.287(0.2759) | -1.879(0.7789) | -1.592(0.8299) | 0.887 | 0.033 | 0.025 |
| Csf2 | -0.1004(0.177) | -0.087(0.2438) | D,2187(0.1565) | -0.009(0.1596) | -0.409(0.2972) | -0.471(0.461) | 0.57 | 0.042 | 0.776 |
| Ccl2 | 0.0429(0.4363) | -0.5385{0.3764} | -0.4959(0.474) | -0.177(0.9251) | -1.498(1.0403) | -1.675(1.484) | 0.962 | 0.0431 | 0.0878 |
| Cxcl1 | 0.3554(0.3142) | -1.7778{0.5756} | 1.4225(0.7439) | -0.591(1.0402) | -2.715(1.1859) | -3.305(1.3832) | 0.161 | 0.193 | 0.00967 |
| ***Product 3*** | | | | | | | | | |
| Ccl20 | 0.2731(0.549) | 0.1061(0.3342) | 0.3793(0.5802) | 0.236(0.6359) | -0.959(0.8818) | -0.723(0.9357) | 0.809 | 0.00152 | 0.00619 |
| Cxcl1 | 0.3554(0.3142) | -1.7778(0.5756) | 1.4225(0.7439) | -0.914(0.5257) | -1.775(0.8739) | -2.69(0.8623) | 0.0000113 | 0.918 | 0.00619 |
| Parpl | 0.5088(1.3727) | -0.0224{1.8097} | 0.4864(2.2125) | -0.853(1.1549) | 1.611(1.0905) | 0.758(0.3603) | 0.043 | 0.00795 | 0.973 |
| **Pdgfb** | -0.3271(1.0878) | 0.5783(1.9866) | 0.2512(2.3147) | -1.705(1.2842) | 2.583(1.7054) | 0.877(2.081) | 0.0295 | 0.0101 | 0.282 |
| Ntf3 | -0.0557(0.6429) | 0.2649(0.7048) | 0.2191(0.2477) | 0.229(0.2534) | -0.241(0.3478) | -0.011(0.2758) | 0.282 | 0.0125 | 0.0952 |
| Il17a | 0.3519(0.5937) | -0.9644(0.8592) | 0.6125(0.7647) | 0.404(0.5028) | -2.401(1.5702) | -1.996(1.5433) | 0.654 | 0.0127 | 0.0127 |
| **Cpe** | 0.0646(0.165) | 0.3941(0.3813) | 0.4587(0.3897) | 0.096(0.2479) | 0.776(0.3635) | 0.872(0.4707) | 0.863 | 0.0295 | 0.0513 |
| Tgfb1 | -0.0922(0.3459) | 0.144(0.5013) | 0.0518(0.5602) | -0.353(0.3248) | 0.548(0.3366) | 0.195(0.3014) | 0.197 | 0.0357 | 0.605 |
| Il10 | 0.202(0.1156) | -0.1221(0.2107) | 0.0676(0.2792) | 0.129(0.2199) | -0.374(0.2464) | -0.246(0.2339) | | 1 0.0379 | 0.272 |
| Tgfa | 0.2167(0.3508) | 0.3138(0.3719) | 0.5306(0.3706) | 0.24(0.4501) | 0.754(0.5729) | 0.994(0.7126) | 0.809 | 0.043 | 0.043 |
| ***Product 4*** | | | | | | | | | |
| Ntf3 | -0.0557(0.6429) | 0.2649(0.7048) | 0.2191(0.2477) | 0.302(0.37) | -0.354(0.2313) | -0.052(0.327) | 0.0789 | 0.00276 | 0.0503 |
| Ccl20 | 0.2731(0.549) | 0.1061(0.3342) | 0.3793(0.5802) | 1.243(1.0697) | -0.889(0.9684) | 0.355(1.1177) | 0.0435 | 0.00762 | 0.905 |

(continued)

| | | | | | | | | | |
|---|---|---|---|---|---|---|---|---|---|
| ***Product 4*** | | | | | | | | | |
| Il17a | 0.3519(0.5937) | -0.9644(0.8592) | 0.6125(0.7647) | 0.028(0.5497) | -2.353(1.4588) | -2.324(1.2537) | 0.447 | 0.0101 | 0.0021 |
| Ccl3 | -0.0815(0.3562) | -0.2533(0.9073) | 0.3348(0.9737) | 0.04(0.4079) | -1.117(1.1079) | -1.077(1.0533) | 0.78 | 0.0133 | 0.0535 |
| Tnfrsf11b | 0.1277(0.3085) | -0.0803(0.2906) | 0.0473(0.1606) | 0.179(0.2836) | -0.672(0.5956) | -0.493(0.5529) | 0.497 | 0.0172 | 0.00414 |
| Plxna4 | 0.0382(0.5244) | 0.1329(0.7193) | 0.2655(0.6473) | 0.525(1.1502) | -0.658(0.4796) | -0.133(1.1186) | 0.536 | 0.0401 | 0.383 |
| Fst | -0.077(0.3399) | 0.4464(0.547) | 0.3693(0.6359) | 0.23(0.512) | 0.011(0.5184) | 0.241(0.329) | 0.315 | 0.0435 | 0.549 |
| Cxcl1 | 0.3554(0.3142) | -1.7778(0.5756) | 1.4225(0.7439) | -0.369(1.0303) | -2.567(1.1638) | -2.936(0.6729) | 0.156 | 0.243 | 0.00065 |

Panel: Olink Mouse Exploratory panel consisting of 92 markers.
Values below lower detection limit of detection (LLOD) were excluded from analysis.
Marker with more than 30% of samples being below LLOD were removed.

In conclusion: The four synbiotic compositions influence three important immune markers in the same way or more as mesalamine, a substance established for the treatment of IBD. This indicates that the synbiotic compositions have a potential for treatment and/or alleviation of IBD. One of the synbiotic products (no 3 equivalent to composition 47) increased the amount of Il10 in plasma. Il 10 has important strong immune suppression properties which is important for treatment of inflammatory diseases such as IBD. This once again shows that the synbiotic composition can have an important role in treatment of IBD.

***User survey***

93 individuals (both men and women, from 18 years old) who had used a synbiotic composition not within the scope of the claims (corresponding to Composition No. 19 in Table 1) for 4 weeks, sporadically up to every day, were questioned about their experiences and perceived effects of intake of the composition on effects concerning the gut/IBD and on infection and inflammation. The composition comprised *Lactobacillus plantarum* (LMG P-20606), *Lactobacillus paracasei* (LMG P-17806), *Pediococcus pentosaceus* (LMG P-20608), and *Leuconostoc. mesenteroides* (LMG P-20607) (total amount of of bacteria >$1,5\times10^{10}$ CFU/dose). Each dose further comprised 3,75 g of fibers (inulin, pectine, resistant starch and beta-glucan). Each individual independently reported any experienced positive effect(s) as well as any negative effects. None reported severe adverse effects or that medical treatment was needed.

*IBD*

6 individuals reported that they suffered from IBD, e.g. Crohn's disease, ulcerative colitis or other intestinal diseases. 4 of these respondents had used the synbiotic composition daily, almost every day or about half of the days. Responses to the question whether they felt that the synbiotic composition had helped them with problems related to the gastrointestinal tract (GI-tract) are shown in Table 5b.

As can be seen in Table 5b, already when the time period of regular intake of a synbiotic composition is as short as 4 weeks, the respondents report an improvement in symptoms related to IBD. In conclusion: the results indicate that a synbiotic composition including *Lactobacillus plantarum* (LMG P-20606), *Lactobacillus paracasei* (LMG P-17806), *Pediococcus pentosaceus* (LMG P-20608), and *Leuconostoc. mesenteroides* (LMG P-20607) and the fibres inulin, pectine, resistant starch and beta-glucan may reduce problems with these symptoms and can be used in the treatment of IBD, including ulcerative colitis and Crohn's disease.

***Table 5b.*** *Number and percentage of individuals (out of 4 individuals) who reported effects after 4 weeks of intake of a synbiotic composition (corresponding to Composition No. 19 in Table 1).*

| **Problem** | **Do you feel that the synbiotic composition helped you with problems with *[problem]?*** | | |
|---|---|---|---|
| | **Yes, absolutely** | **Yes, I get some relief** | **Total percentage that perceived an effect** |
| **General stomach-problems** | 1 (25%) | 3 (75%) | 100% |
| **Pain or discomfort in the abdomen** | 1 (25%) | 2 (50%) | 75% |
| **Constipation or diarrhea** | 1 (25%) | 2 (50%) | 75% |
| **Inflammation of the intestine** | 0 (0%) | 3 (75%) | 75% |
| **Flatulence / gas in the stomach** | 0 (0%) | 3 (75%) | 75% |
| **Often urgently need to get to the toilet to empty the bowel** | 1 (25%) | 1 (25%) | 50% |
| **Mucus or blood in the stool** | 0 (0%) | 1 (25%) | 25% |

**Claims**

**1.** A synbiotic composition for use in the treatment, prevention, amelioration, or reductions of symptoms of Inflammatory Bowel Disease (IBD) in an individual, wherein the synbiotic composition comprises:

- a *Lactobacillus paracasei* strain, wherein the *Lactobacillus paracasei* strain is *Lactobacillus paracasei* LMG P-17806 or *Lactobacillus paracasei* LMG P-26118,

- *Lactobacillus plantarum* LMG P-20606,
- *Bifidobacterium breve* LMG-P-26117,
- *Pediococcus pentosaceus* LMG P-20608, and
- *Leuconostoc mesenteroides* LMG P-20607;

and
at least one dietary fiber chosen from the group consisting of

- inulin,
- pectin,
- beta-glucan,
- resistant starch,
- a galacto-oligosaccharide,
- an isomalto-oligosaccharide, and
- a rice fiber.

**2.** The synbiotic composition for use according to claim 1, wherein the *Lactobacillus paracasei* strain is *Lactobacillus paracasei* LMG P-17806.

**3.** The synbiotic composition for use according to any one of the preceding claims, wherein the composition comprises inulin, pectin, beta-glucan and/or resistant starch.

**4.** The synbiotic composition for use according to any one of the preceding claims, wherein the composition comprises resistant starch, a galacto-oligosaccharide, and/or an isomalto-oligosaccharide.

**5.** The synbiotic composition for use according to any one of the preceding claims, wherein the composition comprises resistant starch.

**6.** The synbiotic composition for use according to claim 5, wherein the composition further comprises inulin, pectin, and beta-glucan.

**7.** The synbiotic composition for use according to any one of the preceding claims, wherein the total amount of bacteria in one dose is $1\times10^6$ to $1\times10^{13}$, such as $5\times10^6$ to $1\times10^{13}$, such as $1\times10^7$ to $1\times10^{13}$, such as $5\times10^7$ to $1\times10^{13}$, such as $1\times10^8$ to $1\times10^{13}$, such as $5\times10^8$ to $5\times10^{12}$, especially $1\times10^9$ to $1\times10^{12}$, such as $5\times10^9$ to $9\times10^{11}$, such as $1\times10^{10}$ to $8\times10^{11}$, such as $5\times10^{10}$ to $7\times10^{11}$, such as $1\times10^{11}$ to $6\times10^{11}$, such as $3\times10^{11}$ to $5\times10^{11}$, such as $4\times10^{11}$ CFUs.

**8.** The synbiotic composition for use according to any one of the preceding claims, wherein the total amount of fibers in one dose is between 0.1 to 20 g, such as 0.5 to 20 g, such as 1 to 20 g, such as 2 to 15 g, such as 2.5 to 12.5 g, such as 5 to 10 g, such as 6 to 8.5 g, such as 7.5 g.

**9.** The synbiotic composition for use according to any one of the preceding claims, wherein the Inflammatory Bowel Disease (IBD) is ulcerative colitis or Crohn's disease.

**10.** The synbiotic composition for use according to any one of the preceding claims, wherein the synbiotic composition is administered orally or rectally or by tube feeding.

**11.** The synbiotic composition for use according to any one of the preceding claims, wherein the synbiotic composition is administered as one dose 1 to 3 times a day, 1 to 7 times a week.

**12.** The synbiotic composition for use according to any one of the preceding claims, wherein the synbiotic composition is in the form of a powder, a capsule, a tablet, a lozenge, a liquid, an emulsion, an enema, a suppository, or a tube feeding.

**13.** The synbiotic composition for use according to any one of the preceding claims, wherein the synbiotic composition is a food supplement, a food product, a nutritional supplement, a natural remedy or a pharmaceutical product.

## Patentansprüche

1. Synbiotische Zusammensetzung zur Verwendung bei der Behandlung, Prävention, Verbesserung oder Verringerung von Symptomen von entzündlicher Darmerkrankung (IBD) bei einem Individuum, wobei die synbiotische Zusammensetzung umfasst:

    - einen *Lactobacillus paracasei*-Stamm, wobei der *Lactobacillus paracasei*-Stamm *Lactobacillus paracasei* LMG P-17806 oder *Lactobacillus paracasei* LMG P-26118 ist,
    - *Lactobacillus plantarum* LMG P-20606,
    - *Bifidobacterium breve* LMG P-26117,
    - *Pediococcus pentosaceus* LMG P-20608 und
    - *Leuconostoc mesenteroides* LMG P-20607;

    und
    wenigstens einen Ballaststoff, ausgewählt aus der Gruppe bestehend aus

    - Inulin,
    - Pectin,
    - Beta-Glucan,
    - resistenter Stärke,
    - einem Galacto-Oligosaccharid,
    - einem Isomalto-Oligosaccharid und
    - einer Reisfaser.

2. Synbiotische Zusammensetzung zur Verwendung nach Anspruch 1, wobei der *Lactobacillus paracasei*-Stamm *Lactobacillus paracasei* LMG P-17806 ist.

3. Synbiotische Zusammensetzung zur Verwendung nach einem der vorangehenden Ansprüche, wobei die Zusammensetzung Inulin, Pectin, Beta-Glucan und/oder resistente Stärke umfasst.

4. Synbiotische Zusammensetzung zur Verwendung nach einem der vorangehenden Ansprüche, wobei die Zusammensetzung resistente Stärke, ein Galacto-Oligosaccharid und/oder ein Isomalto-Oligosaccharid umfasst.

5. Synbiotische Zusammensetzung zur Verwendung nach einem der vorangehenden Ansprüche, wobei die Zusammensetzung resistente Stärke umfasst.

6. Synbiotische Zusammensetzung zur Verwendung nach Anspruch 5, wobei die Zusammensetzung weiter Inulin, Pectin und Beta-Glucan umfasst.

7. Synbiotische Zusammensetzung zur Verwendung nach einem der vorangehenden Ansprüche, wobei die Gesamtmenge an Bakterien in einer Dosis $1\times10^{6}$ bis $1\times10^{13}$ beträgt, wie etwa $5\times10^{6}$ bis $1\times10^{13}$, wie etwa $1\times10^{7}$ bis $1\times10^{13}$, wie etwa $5\times10^{7}$ bis $1\times10^{13}$, wie etwa $1\times10^{8}$ bis $1\times10^{13}$, wie etwa $5\times10^{8}$ bis $5\times10^{12}$, insbesondere $1\times10^{9}$ bis $1\times10^{12}$, wie etwa $5\times10^{9}$ bis $9\times10^{11}$, wie etwa $1\times10^{10}$ bis $8\times10^{11}$, wie etwa $5\times10^{10}$ bis $7\times10^{11}$, wie etwa $1\times10^{11}$ bis $6\times10^{11}$, wie etwa $3\times10^{11}$ bis $5\times10^{11}$, wie etwa $4\times10^{11}$ KBEs.

8. Synbiotische Zusammensetzung zur Verwendung nach einem der vorangehenden Ansprüche, wobei die Gesamtmenge an Ballaststoffen in einer Dosis zwischen 0,1 bis 20 g beträgt, wie etwa 0,5 bis 20 g, wie etwa 1 bis 20 g, wie etwa 2 bis 15 g, wie etwa 2,5 bis 12,5 g, wie etwa 5 bis 10 g, wie etwa 6 bis 8,5 g, wie etwa 7,5 g.

9. Synbiotische Zusammensetzung zur Verwendung nach einem der vorangehenden Ansprüche, wobei die entzündliche Darmerkrankung (IBD) Colitis ulcerosa oder Morbus Crohn ist.

10. Synbiotische Zusammensetzung zur Verwendung nach einem der vorangehenden Ansprüche, wobei die synbiotische Zusammensetzung oral oder rektal oder durch Sondenernährung verabreicht wird.

11. Synbiotische Zusammensetzung zur Verwendung nach einem der vorangehenden Ansprüche, wobei die synbiotische Zusammensetzung als eine Dosis 1- bis 3-mal am Tag, 1- bis 7-mal in der Woche verabreicht wird.

**12.** Synbiotische Zusammensetzung zur Verwendung nach einem der vorangehenden Ansprüche, wobei die synbiotische Zusammensetzung in der Form eines Pulvers, einer Kapsel, einer Tablette, einer Pastille, einer Flüssigkeit, einer Emulsion, eines Klistiers, eines Suppositoriums oder einer Sondenernährung vorliegt.

**13.** Synbiotische Zusammensetzung zur Verwendung nach einem der vorangehenden Ansprüche, wobei die synbiotische Zusammensetzung ein Nahrungsmittelergänzungsstoff, ein Nahrungsmittelprodukt, eine Nährstoffergänzungsmittel, ein natürliches Heilmittel oder ein pharmazeutisches Produkt ist.

## Revendications

**1.** Composition synbiotique destinée à être utilisée dans le traitement, la prévention, l'amélioration ou la réduction de symptômes de maladie inflammatoire de l'intestin (MII) chez un individu, dans laquelle la composition synbiotique comprend :

- une souche de *Lactobacillus paracasei*, dans laquelle la souche de *Lactobacillus paracasei* est *Lactobacillus paracasei* LMG P-17806 ou *Lactobacillus paracasei* LMG P-26118,
- *Lactobacillus plantarum* LMG P-20606,
- *Bifidobacterium breve* LMG P-26117,
- *Pediococcus pentosaceus* LMG P-20608, et
- *Leuconostoc mesenteroides* LMG P-20607 ;

et
au moins une fibre alimentaire choisie dans le groupe constitué de

- l'inuline,
- la pectine,
- le bêta-glucane,
- l'amidon résistant,
- un galacto-oligosaccharide,
- un isomalto-oligosaccharide, et
- une fibre de riz.

**2.** Composition synbiotique destinée à être utilisée selon la revendication 1, dans laquelle la souche *Lactobacillus paracasei* est *Lactobacillus paracasei* LMG P-17806.

**3.** Composition synbiotique destinée à être utilisée selon l'une quelconque des revendications précédentes, dans laquelle la composition comprend de l'inuline, de la pectine, du bêta-glucane et/ou de l'amidon résistant.

**4.** Composition synbiotique destinée à être utilisée selon l'une quelconque des revendications précédentes, dans laquelle la composition comprend de l'amidon résistant, un galacto-oligosaccharide et/ou un isomalto-oligosaccharide.

**5.** Composition synbiotique destinée à être utilisée selon l'une quelconque des revendications précédentes, dans laquelle la composition comprend de l'amidon résistant.

**6.** Composition synbiotique destinée à être utilisée selon la revendication 5, dans laquelle la composition comprend en outre de l'inuline, de la pectine et du bêta-glucane.

**7.** Composition synbiotique destinée à être utilisée selon l'une quelconque des revendications précédentes, dans laquelle la quantité totale de bactéries dans une dose est de $1 \times \times 10^6$ à $1 \times 10^{13}$, telle que $5 \times 10^6$ à $1 \times 10^{13}$, telle que $1 \times 10^7$ à $1 \times 10^{13}$, telle que $5 \times 10^7$ à $1 \times 10^{13}$, telle que $1 \times 10^8$ à $1 \times 10^{13}$, telle que $5 \times 10^8$ à $5 \times 10^{12}$, notamment $1 \times 10^9$ à $1 \times 10^{12}$, telle que $5 \times 10^9$ à $9 \times 10^{11}$, telle que $1 \times 10^{10}$ à $8 \times 10^{11}$, telle que $5 \times 10^{10}$ à $7 \times 10^{11}$, telle que $1 \times 10^{11}$ à $6 \times 10^{11}$, telle que $3 \times 10^{11}$ à $5 \times 10^{11}$, telle que $4 \times 10^{11}$ UFC.

**8.** Composition synbiotique destinée à être utilisée selon l'une quelconque des revendications précédentes, dans laquelle la quantité totale de fibres dans une dose est entre 0,1 et 20 g, telle que 0,5 et 20 g, telle que 1 et 20 g, telle que 2 et 15 g, telle que 2,5 et 12,5 g, telle que 5 et 10 g, telle que 6 et 8,5 g, telle que 7,5 g.

**9.** Composition synbiotique destinée à être utilisée selon l'une quelconque des revendications précédentes, dans laquelle la maladie inflammatoire de l'intestin (MII) est la rectocolite ulcéreuse ou la maladie de Crohn.

**10.** Composition synbiotique destinée à être utilisée selon l'une quelconque des revendications précédentes, dans laquelle la composition synbiotique est administrée par voie orale ou rectale ou par alimentation par sonde.

**11.** Composition synbiotique destinée à être utilisée selon l'une quelconque des revendications précédentes, dans laquelle la composition synbiotique est administrée en une dose 1 à 3 fois par jour, 1 à 7 fois par semaine.

**12.** Composition synbiotique destinée à être utilisée selon l'une quelconque des revendications précédentes, dans laquelle la composition synbiotique se présente sous la forme d'une poudre, d'une capsule, d'un comprimé, d'une pastille, d'un liquide, d'une émulsion, d'un lavement, d'un suppositoire ou d'une sonde d'alimentation.

**13.** Composition synbiotique destinée à être utilisée selon l'une quelconque des revendications précédentes, dans laquelle la composition synbiotique est un complément alimentaire, un produit alimentaire, un complément nutritionnel, un remède naturel ou un produit pharmaceutique.

Fig. 1a

1%DSS in drinking water
Day -7
Day -3
Day 0
Day 4
Day 14
END
IBD-induction
Treatment period
Adaptation
Start
Blood
AWR1
Blood,
AWR 2
Blood
AWR3

Fig. 1b

Histological Score
30
20
10
0
a
bc
b
ac
b
ac
b
Intact
#37
#56
#47
#50
MEZ
Placebo

Fig. 1c

Ccl20
10.0
9.5
9.0
8.5
t1
t2
t3
Timepoint
group
Mesalamine
Placebo
Product 3

Fig. 1d

Cxcl1
12
11
10
9
t1
t2
t3
Timepoint
group
Mesalamine
Placebo
Product 3

Fig. 1e

3.0
2.8
2.6
2.4
Il10
t1
t2
t3
Timepoint
group
Mesalamine
Placebo
Product 3


Fig. 1f

6
5
4
3
2
Il17a
t1
t2
t3
Timepoint
group
Mesalamine
Placebo
Product 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2005077391 A1
- US 9579353 B2
- EP 1624762 B1
- EP 2672980 B1